# EUROPÄISCHE PATENTSCHRIFT

(11) **EP 0 625 351 B1**
(45) Veröffentlichungstag und Bekanntmachung des Hinweises auf die Patenterteilung: **07.01.1999**
(21) Anmeldenummer: 94106819.9
(22) Anmeldetag: 02.05.1994
(51) Int. Cl.: A61K 31/44

(54) **Verwendung von Etofibrat und pharmazeutische Zusammensetzungen, enthaltend Etofibrat, zur Behandlung der diabetischen Angio- und Retinopathie**
Use of Etofibrate and pharmaceutical compositions containing etofibrate in the treatment of diabetic angio- and retinopathy
Utilisation de l'étofibrate et des compositions/pharmaceutiques contenant de l'étofibrate pour le traitement de l'angiopathie et rétinopathie diabétiques

(30) Priorität: 19.05.1993 DE 4317127
(43) Veröffentlichungstag der Anmeldung: 23.11.1994
(73) Patentinhaber: MERZ + CO. GmbH & Co., 60318 Frankfurt (DE)
(72) Erfinder: Erbler, Hans, Dr. med., D-42115 Wuppertal (DE)
(74) Vertreter: Beil, Hans Chr., Dr.

(56) Entgegenhaltungen:
- EP-A- 0 168 360
- DE-A- 1 941 217
- DE-A- 3 621 861
- DE-A- 4 021 678
- MUNCH. MED. WOCHENSCHR. Bd. 135, Nr. 42 , 22. Oktober 1993 Seite 54 'Lipidsenker gegen diabetische Retinopathie?'
- FORTSCHR.MED. Bd. 112, Nr. 3 , 30. Januar 1994 Seiten 45 - 46 K.-W-STOCKERT 'Fibrate senken auch Fibrinogenspiegel'
- FORTSCHR.MED. Bd. 94, Nr. 13 , 6. Mai 1976 Seiten 785 - 790 J.SCHNEIDER ET AL. 'Feldstudie zur Lipidsenkung mit Etofibrat'
- THERAPIEWOCHE Bd. 34, Nr. 8 , 1984 Seiten 1122 - 1129 H.S.BADRAWY ET AL. 'Einfluss von Etofibrat auf die Blutglukose, Cholesterin und Triglyzeride'
- PHARMACOL.RES. Bd. 26, Nr. 3 , 1992 Seiten 243 - 260 C.R.SIRTORI ET AL. 'TOLERABILITY OF FIBRIC ACIDS. COMPARATIVE DATA AND BIOCHEMICAL BASES'

## Beschreibung

Die vorliegende Erfindung betrifft die Verwendung einer pharmazeutischen Zusammensetzung, enthaltend Etofibrat in einer wirksamen Dosis, zur Behandlung der diabetischen Angiopathie, insbesondere der diabetischen Retinopathie. Zusammensetzungen, enthaltend Etofibrat, in Form von Pellets mit einem besonderen Überzug aus Polyvinylalkohol und einem Sprengmittel sowie Cellulose, sind besonders geeignet für die genannte Indikation.

Bei etwa einem Drittel der Patienten mit Diabetes mellitus entwickelt sich im Krankheitsverlauf eine diabetische Angiopathie, insbesondere eine Retinopathie (Mehnert und Schöffling; Diabetologie in Klinik und Praxis; 2. Auflage, Georg-Thieme-Verlag Stuttgart, New York, 1984). Die Retinopathie korreliert u.a. mit der diabetischen Stoffwechsellage, hohen Fibrinogenspiegeln und Veränderungen der Mikrozirkulation durch Erhöhung der Plasmaviskosität.

Aufgabe vorliegender Erfindung ist es, ein Mittel bereitzustellen, das sich zur Behandlung der diabetischen Angiopathie, insbesondere der diabetischen Retinopathie eignet, welches gut verträglich ist und geringe Nebenwirkungen aufweist.

Diese Aufgabe wird erfindungsgemäß gelöst durch die Verwendung einer pharmazeutischen Zusammensetzung gemäß Anspruch 1, enthaltend Etofibrat als Wirkstoff. Dieser kann bevorzugt in Mengen von 500-1000 mg/Einheit in Form von Kapseln oder Tabletten als Suspension oder Emulsion mit üblichen Hilfs- und Zusatzstoffen eingesetzt werden.

Etofibrat weist bekanntlich eine Cholesterin-senkende und HDL-Cholesterin-erhöhende Wirkung sowie eine Triglyceridsenkende Wirkung und eine Absenkung der Fibrinogenspiegel und der Plasmaviskosität bei der Behandlung von Hyperlipoproteinämie auf.

Aus DE-A-36 21 861 ist die Herstellung eines Etofibrat-haltigen Arzneimittels zur Behandlung unter anderem von Necrobiosis lipoidica diabeticorum bekannt. Eine Verwendung dieses Mittels zur Behandlung anderer diabetischer Angiopathien ist dort weder beschrieben noch liegt sie für den Fachmann nur nahe.

Überraschenderweise wurde nun gefunden, daß der Wirkstoff Etofibrat auch für die Behandlung anderer diabetischer Angiopathien und insbesondere der diabetischen Retinopathie geeignet ist.

In einer klinischen Studie konnte bei Patienten mit Diabetes mellitus, bei welchen eine beginnende nicht-proliferative Retinopathie vorlag, eine Rückbildung der Exsudate der Augen sowie eine Absenkung der Triglyceridwerte und der hohen Fibrinogenspiegel festgestellt werden.

Dabei wird Etofibrat therapeutisch in einer Dosierung zwischen 300 und 1000 mg, insbesondere 500-1000 mg, gegebenenfalls bis zu 1500 mg pro Tag appliziert. Je nach Gesamtdosis erfolgt die Verabreichung der Dosiseinheiten abends, morgens und abends oder 3 x täglich.

Erfindungsgemäß wird Etofibrat vorzugsweise oral verabreicht. Als Mittel zur oralen Verabreichung kommen feste Formen, beispielsweise Tabletten und Kapseln und flüssige Formen, beispielsweise ölige Suspensionen, Emulsionen oder liposomale Zubereitungen in Kapseln in Frage.

Bevorzugte orale feste Mittel liegen in der Form von Tabletten und Kapseln vor und können übliche Träger wie Bindemittel (z.B. Gelatine, Sorbit, Polyvinylpyrrolidon, Cellulosederivate, wie z.B. Methylcellulose, Hydroxypropylmethylcellulose) Füllstoffe (z.B. Lactose, Stärke, Calciumphosphat, Sorbit, Cellulosederivate), Gleitmittel (z.B. Magnesiumstearat, Talk, Polyethylenglykol oder Silicumdioxid), Disintegrationsmittel (z.B. quervernetztes PVP) und Netzmittel (z.B. Polysorbate) enthalten. Diese Komponenten sind dem Fachmann bekannt und können je nach Bedarf leicht ausgewählt werden.

Die Herstellung der obengenannten Etofibrat enthaltenden Arzneimittelformulierungen sind dem Fachmann bekannt.

Ganz besonders bevorzugt wird eine pharmazeutische Zusammensetzung, welche Etofibrat in der angegebenen Menge in Form von Pellet-Tabletten enthält, wobei die Pellets einen Überzug aus einem teilverseiften Polyvinylalkohol, einem Sprengmittel, wie z.B. Polyvinylpyrrolidon, Cellulose und gegebenenfalls weiteren Kolloiden sowie Farb- und/oder Aromastoffen aufweisen. Eine derartige Formulierung ist besonders stabil und für die angegebene Indikation besonders geeignet.

Die Erfindung wird anhand der nachfolgenden Beispiele näher erläutert.

### I. Herstellung der pharmazeutischen Zusammensetzungen

### 1. Erfindungsgemäße Zusammenstzung, enthaltend Etofibrat in Form von Pellet-Tabletten

| | |
|---|---|
| Kapselinhalt, pelletisiert | 1000,00 mg |
| Etofibrat | 750,00 mg |
| Guar-Gummi (Galactomannan 60) | 58,00 mg |
| Eudragit RS 30 D | 216,00 mg |
| Triacetin | 6,00 mg |
| Chinolingelb (E 104) | 0,20 mg |
| Polyvinylalkohol (teilverseift) | 27,00 mg |
| Poly(1-vinyl-2-pyrrolidon) ringöffnend vernetzt | 36,40 mg |
| Cellulosepulver | 35,70 mg |
| Saccharin-Na-2H₂O | 4,30 mg |
| Grapefruit-Aroma | 5,40 mg |
| Macrogel 6000 | 11,30 mg |
| Hochdisperses Siliciumdioxid | 0,70 mg |

### Herstellungsvorschrift

### Herstellung der Extrusionspellets:

In einem geeigneten Mischgranulierer werden das gesiebte Etofibrat sowie Galactomannan 60 trocken gemischt. In einem geeigneten Rührmischer wird aus 90% des Chinolingelbanteils, 8,2 kg Wasser, Triacetin und 26 kg Eudragit RS 30 D eine homogene Dispersion hergestellt. Mit dieser Dispersion wird die Mischung aus Etofibrat und Galactomannan granuliert. Das Granulat wird anschließend extrudiert.

Die Extrusionsformkörper werden in einem geeigneten Spheronizer ausgerundet und getrocknet.

### Herstellen der Überzugssuspension:

In einem Mischer-Homogenisator wird der Polyvinylalkohol in 74,5 kg Wasser gelöst. Saccharin-Na, Poly(1-vinyl-2-pyrrolidon), Cellulosepulver, hochdisperses Siliciumdioxid und der Rest Chinolingelb werden eingetragen und homogenisiert. Zuletzt wird das Grapefruit-Aroma eingetragen.

### Coaten der Pellets:

Die Extrusionspellets werden in einem Wirbelschichtgranulator mittels einer Zweistoffdüse mit der Überzugsdispersion besprüht. Nach Beendigung des Suspensionsauftrages wird unter den gleichen Bedingungen die Lösung von Macrogol in 3,1 kg H₂O aufgesprüht und getrocknet.

### Tablettierung:

Die gecoateten Pellets werden auf einer geeigneten Rundläuferpresse unter Preßkraftüberwachung zu Oblong-Tabletten von 1000 mg Gewicht 2 (= 750 mg Etofibrat) verpreßt.

### 2. Etofibrat, appliziert als Weichgelatinekapsel

| | |
|---|---|
| Kapselinhalt | 580,00 mg |
| Etofibrat | 300,00 mg |
| Miglyol 812 | 236,00 mg |
| Wachsmischung | 40,00 mg |
| Sojalecithin | 4,00 mg |
| magensaftresistenz überzogene Kapselhülle: | 290,00 mg |
| Gesamtgewicht der Kapseln: | 870,00 mg |

### Herstellungsvorschrift

### Ansatz des Kapselfüllgutes:

Etofibrat wird in der Wachs-Öl-Mischung suspendiert. Nach Mahlen, Sieben und Entlüften der Masse wird die Suspension der Füllmaschine zugeführt.

### Verkapselung:

Aus der temperierten Gelatinemasse werden an der Verkapselungsmaschine zwei Endlosbänder geformt und zwischen zwei gegeneinander routierender Formwalzen geleitet. Aus diesen Gelatinebändern werden die Wandelemente der Kapsel durch den Walzendruck als Halbform ausgestanzt und an ihren Rändern miteinander verschweißt, während synchron eine Dosierpumpe das Füllgut mit Hilfe eines Füllkeils zwischen diesen beiden Wandelemente preßt. Dabei erhalten diese Kapseln ihre endgültige Form.

### Lackierung:

Nach der Auftrocknung der Kapseln bei Raumtemperatur durch klimatisierte Luft werden die Kapseln in einer Wirbelschichtapparatur mit einem magensaftresistenten Lack beschichtet.

### 3. Etofibrat als Retard-Formulierung in Hartgelatinekapseln

| | |
|---|---|
| Kapselinhalt | 518,15 mg |
| Etofibrat | 500,00 mg |
| Zucker | 4,50 mg |
| Maisstärke | 1,50 mg |
| Talkum | 6,40 mg |
| Schellack | 5,75 mg |
| Kapselhülle: | 97,00 mg |
| Gesamtgewicht der Kapsel: | 615,15 mg |

### Herstellungsvorschrift:

Die Herstellung erfolgt durch kontinuierlichen Aufbau über die Stufen Kerngranulat und Pellets.

### Kerngranulat:

Saccharose (3,15 kg) und Maisstärke (1,05 kg) werden in einem geeigneten Kessel gemischt. Durch Auftragen einer Lösung aus 50 kg Etofibrat und 1 kg Schellack in 14,5%iger Lösung in Ethanol wird ein Kerngranulat mit einer Korngröße zwischen 125-800 µm, insbesondere 320-630 µm hergestellt.

### Pellets:

Auf 55,2 kg Kerngranulat wird eine Lösung von 300 kg Etofibrat in 30 kg Ethanol kontinuierlich aufgetragen. Es entstehen Pellets mit einem Durchmesser zwischen 630-1250 µm. Auf die Pellets wird Schellack in 40%iger Lösung in Ethanol aufgesprüht und mit Talkum abgestreut.

### Kapseln:

Die Pellets werden auf einer Kapselfüllmaschine in Hartgelatinekapseln (Größe 0) gefüllt (entsprechend 500 mg Etofibrat pro Kapsel)

### II. Behandlung von Patienten mit diabetischer Retinopathie

### a) Ablauf der klinischen Studie

Im Rahmen einer Pilotstudie wurden 8 Patienten mit Diabetes mellitus, einer Hyperlipoproteinämie (Gesamt-Cholesterin größer/gleich 200 mg/dl und kleiner als 400 mg/dl und Triglyceride größer/gleich 200 mg/dl und kleiner als 500 mg/dl) sowie Zeichen einer beginnenden nicht-proliferativen Retinopathie (Stadium I und II) 6 Monate lang mit 2 x 500 mg Etofibrat pro Tag behandelt. Zu Beginn der Untersuchung, nach 2, 4 und 6 Monaten wurden die Patienten auf ihre Krankheitserscheinungen hin untersucht. An diesen Untersuchungsterminen erfolgte eine Diabeteskontrolle (Glukose, Glukoseausscheidung, HbA1). Weiterhin erfolgte die Bestimmung der Lipide (Triglyceride, Cholesterin, LDL-Cholesterin, HDL-Cholesterin), des Fibrinogens sowie von Serumkreatinin, Plasma-Eiweiß und Proteinurie als Nephropathiezeichen. Bei der ersten und der letzten Untersuchung erfolgte die Dokumentation des Augenhintergrundes mittels Funduskamera. Es erfolgte eine Beurteilung des Befundes nach den Kriterien Kaliberschwankungen, Exsudate, Mikroaneurysmen, Gefäßproliferationen, Blutungen.

### b) Ergebnisse

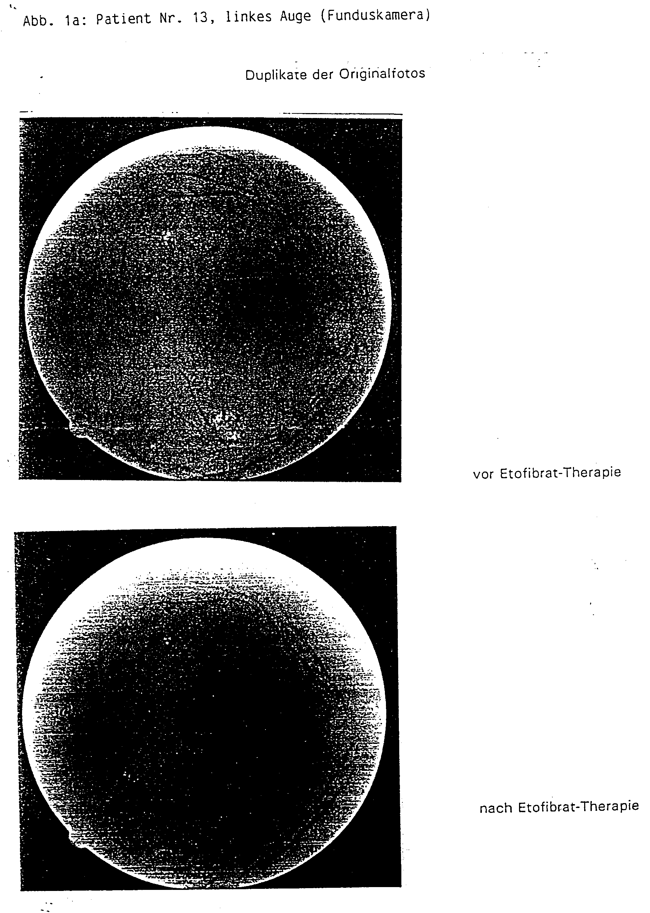

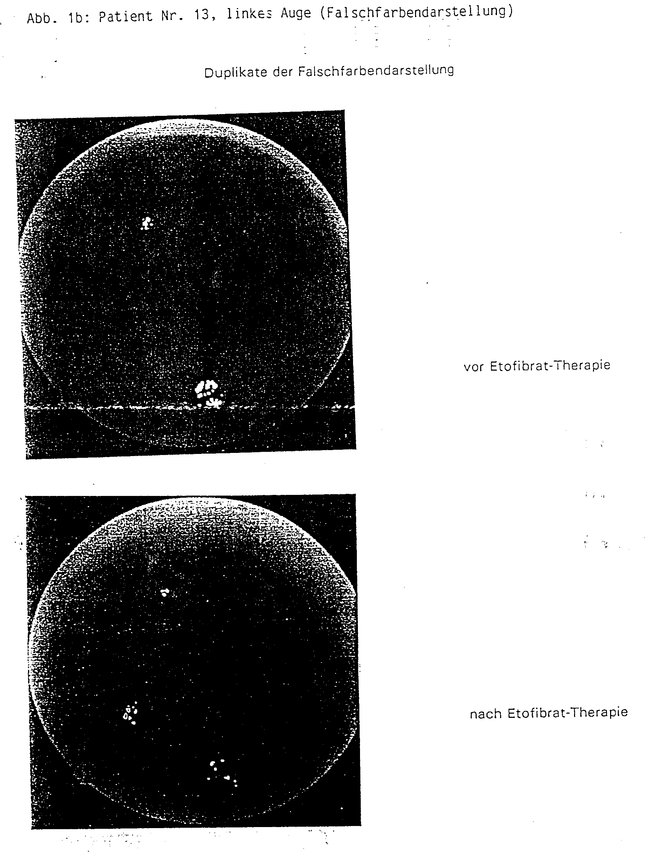

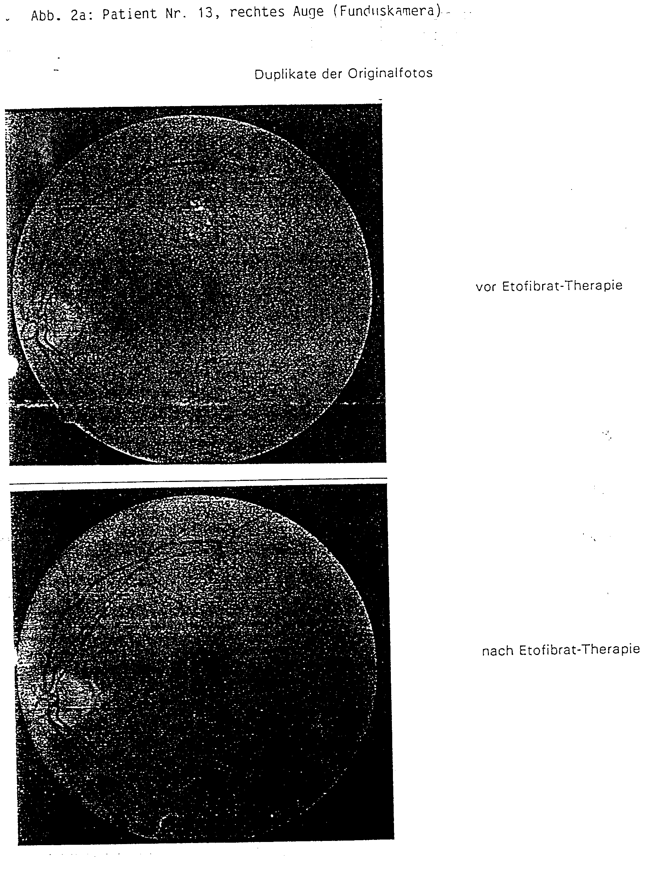

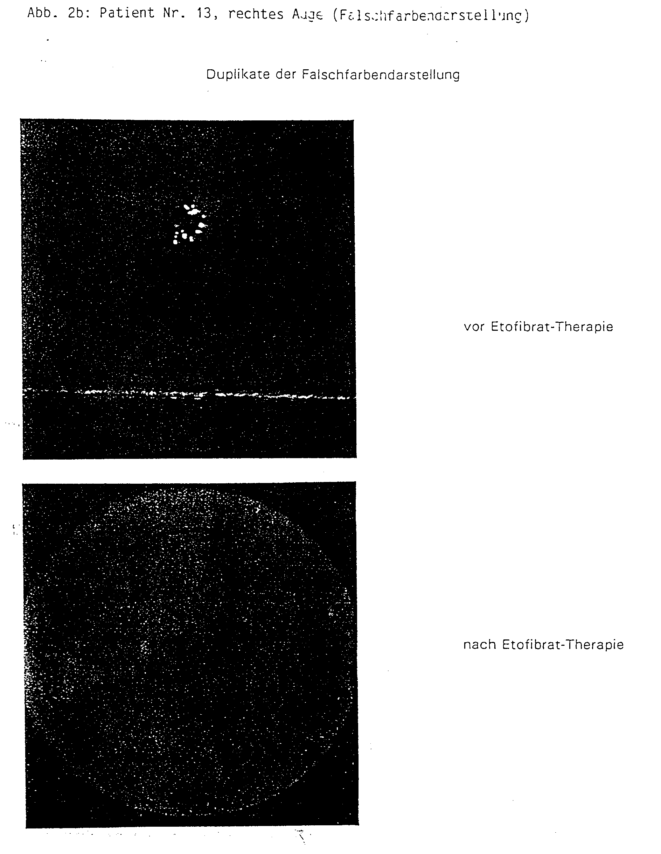

Bei 7 von 8 Patienten war eine deutliche Rückbildung der mittels Funduskamera dokumentierten Retinopathie nachweisbar. Bei der Beurteilung des Augenhintergrundes fiel insbesondere der Rückgang der harten Exsudate auf. Abbildung 1a zeigt für einen Patienten (Nr. 13) den Augenhintergrund (Funduskamera) des linken Auges vor und nach Etofibrat-Therapie. Die Zurückbildung der Exsudate ist insbesondere auch in der Falschfarbendarstellung dieser Aufnahmen (Abb. 1b) deutlich zu sehen. Auch beim rechten Auge (2a und b) zeigt sich die Rückbildung der Exsudate. Eine teilweise erhebliche Absenkung der Triglyceridwerte war bei 6 von 8 Patienten festzustellen. Ferner wurde bei 6-8 von 8 Patienten der Fibrinogenspiegel deutlich gesenkt.

## Patentansprüche

1. Verwendung einer pharmazeutischen Zusammensetzung, enthaltend eine wirksame Dosis Etofibrat und übliche Träger- und Zusatzstoffe, zur Herstellung eines Medikaments zur Behandlung der diabetischen Angiopathie, jedoch mit Ausnahme von Necrobiosis lipoidica diabeticorum.

2. Verwendung einer Zusammensetzung gemäß Anspruch 1 zur Herstellung eines Medikaments zur Behandlung der diabetischen Retinopathie.

3. Verwendung gemäß Anspruch 1 oder 2, dadurch gekennzeichnet, daß die Zusammensetzung eine wirksame Dosis von 300 bis 1000 mg Etofibrat/Einheit enthält.

4. Verwendung gemäß einem der Ansprüche 1-3, dadurch gekennzeichnet, daß der Wirkstoff in Form von Tabletten oder Kapseln eingesetzt wird.

5. Verwendung gemäß einem der Ansprüche 1-4, dadurch gekennzeichnet, daß als Träger Gelatine, Sorbit, Polyvinylpyrrolidon oder Cellulosederivate und als Zusatzstoffe Füllstoffe, Gleitmittel, Disintegrationsmittel und Netzmittel eingesetzt werden.

6. Verwendung gemäß einem der Ansprüche 1-3, dadurch gekennzeichnet, daß der Wirkstoff in flüssiger Form in Weichgelatinekapseln als Emulsion, Suspension oder liposomale Zubereitungen eingesetzt wird.

## Claims

1. The use of a pharmaceutical composition containing an effective dose of etofibrate and customary carrier and additive substances for the production of a drug for the treatment of diabetic angiopathy, with the exception of necrobiosis lipoidica diabeticorum.

2. The use of a composition according to claim 1 for the production of a drug for the treatment of diabetic retinopathy.

3. A use according to claims 1 or 2, characterised in that the composition contains an effective dose of 300 to 1000 mg etofibrate/unit.

4. A use according to any one of claims 1-3, characterised in that the active ingredient is used in the form of tablets or capsules.

5. A use according to any one of claims 1-4, characterised in that gelatine, sorbitol, polyvinylpyrrolidone or cellulose derivatives are used as the carrier, and fillers, internal lubricants, disintegration agents and wetting agents are used as the additives.

6. A use according to any one of claims 1-3, characterised in that the active ingredient is used in liquid form as an emulsion, as a suspension or as liposomal preparations in soft gelatine capsules.

## Revendications

1. Utilisation d'une composition pharmaceutique, contenant une dose active d'étofibrate et des supports et additifs classiques, pour la préparation d'un médicament destiné au traitement des angiopathies diabétiques, à l'exclusion cependant de la nécrose lipoïdique des diabétiques.

2. Utilisation d'une composition selon la revendication 1 pour la préparation d'un médicament destiné au traitement de la rétinopathie diabétique.

3. Utilisation selon la revendication 1 ou 2, caractérisée en ce que la composition contient une dose active de 300 à 1 000 mg d'étofibrate/unité.

4. Utilisation selon l'une des revendications 1 à 3, caractérisée en ce que la substance active est utilisée sous forme de comprimés ou de capsules.

5. Utilisation selon l'une des revendications 1 à 4, caractérisée en ce que l'on utilise comme supports de la gélatine, du sorbitol, de la polyvinylpyrrolidone ou des dérivés de cellulose, et comme additifs des charges, des lubrifiants, des agents désintégrants et des agents mouillants.

6. Utilisation selon l'une des revendications 1 à 3, caractérisée en ce que l'on utilise la substance active sous forme liquide en tant qu'émulsion, suspension ou préparation en liposomes dans des capsules de gélatine molle.
